Europäisches Patentamt

(19) European Patent Office

Office européen des brevets

(11) Publication number: **0 146 414**
**B1**

(12) **EUROPEAN PATENT SPECIFICATION**

(45) Date of publication of patent specification: **25.10.89**

(21) Application number: **84309044.0**

(22) Date of filing: **21.12.84**

(51) Int. Cl.⁴: **A 61 K 31/71,** A 61 K 47/00

(54) **Antiparasitic non-aqueous formulation containing an avermectin or milbemycin.**

(30) Priority: **22.12.83 US 564140**

(43) Date of publication of application:
**26.06.85 Bulletin 85/26**

(45) Publication of the grant of the patent:
**25.10.89 Bulletin 89/43**

(84) Designated Contracting States:
**AT BE CH DE FR GB IT LI LU NL SE**

(56) References cited:
**EP-A-0 002 916**
**US-A-4 130 651**
**US-A-4 430 329**

(73) Proprietor: **MERCK & CO. INC.**
**126, East Lincoln Avenue P.O. Box 2000**
**Rahway New Jersey 07065-0900 (US)**

(72) Inventor: **Williams, James B.**
**4, Coventry Drive**
**Freehold New Jersey (US)**
Inventor: **Nesbitt, Russell U.**
**292 Miller Avenue**
**Somerville New Jersey 08876 (US)**

(74) Representative: **Crampton, Keith John Allen**
**et al**
**D YOUNG & CO 10 Staple Inn**
**London WC1V 7RD (GB)**

Courier Press, Leamington Spa, England.

## Description

The present invention concerns the preparation of avermectin and milbemycin parenteral formulations. More particularly, but not exclusively, the avermectin is ivermectin, an anthelmintic agent.

The avermectin family, of which ivermectin is a member, is a series of new and very potent antiparasitic agents which are useful against a broad spectrum of endoparasites and ectoparasites in mammals as well as having agricultural uses against various parasites found in and on crops and in soil.

The basic avermection compounds are isolated from the fermentation broth of the microorganism *Streptomyces avermitilis*. Such compounds are described in U.S. Patent US—A—4,310,519. In addition, certain derivatives of these basic fermentation products have been prepared.

Some of the avermectins contain a 22,23-double bond. This may be selectively reduced to prepare the ivermectin compounds. In addition, the avermectins possess a disaccharide residue at the 13-position consisting of the α-L-oleandrosyl-α-L-oleandrosyl group. One or both of these saccharide groups may be removed as described in U.S. Patent US—A—4,206,205. The thus produced aglycone derivatives have a hydroxy group at the 13-position. This group may be removed to form the 13-deoxy compound as described in U.S. Patents US—A—4,171,314 and US—A—4,173,571. On the avermectin compounds and derivatives are several hydroxy groups which may be acylated as described in U.S. Patent US—A—4,201,861.

In addition, the milbemycin series of compounds have the same 16-membered macrocyclic ring as the avermection compounds, although they do not have the disaccharide residue and also differ in the nature of other substituent groups. These compounds are disclosed in U.S. Patent US—A—3,950,360.

Ivermection is disclosed in U.S. Patent US—A—4,199,569, issued 22 April 1980. Ivermectin is a mixture, in the ratio of approximately 80:20 of 22,23-dihydro C-076 Bla and Blb. In administering ivermectin to animals it is very convenient to use parenteral formulations to administer the drug. While an aqueous micelle formulation (see U.S. Patent US—A—4,389,397) is useful for parenteral administration, higher activity has been observed against endoparasites than ectoparasites. It is known that ivermectin can be useful against ectoparasites and it is desirable to have a parenteral formulation with high levels of both endo-and ectoparasiticidal activity.

Thus, it is desirable to prepare a parenteral liquid formulation of ivermectin which has activity against internal and external parasites. Ivermectin has very poor solubility in water, at a level of about 0.005 mg per ml at room temperature, but is very soluble in many organic solvents.

It was unexpectedly discovered during the investigation of organic parenteral formulations that both endo- and ectoparasiticidal activity was observed which was not observed with the aqueous micelle formulation.

The invention is based on the unexpected discovery that the spectrum of activity of avermectin compounds is increased by the use of parenteral formulations described below. The parenternal formulations consist of glycerol formal and propylene glycol together with an avermectin or milbemycin compound. All such compounds share to an only slightly varying degree the spectrum of biological activity of ivermectin, which is the preferred compound.

In the formulations of the present invention, the amount of the avermectin or milbemycin compound is 0.1 to 20 by weight, preferably 1% by weight. The avermectin compound is dissolved in a solvent mixture of glycerol formal and proplyene glycol which are in a ratio of from 10:90 to 90:10 by volume (V/V). It is preferred to use a ratio of 40:60 V/V.

A variation of this formulation replaces the glycerol formal with water at a lower concentration to achieve the same expanded profile of activity. The formulation uses ratios of propylene glycol to water of from 95:5 to 80:20 V/V, preferably 90:10 V/V.

To prepare the formulations the avermectin compound is dissolved in either the glycerol formal or propylene gylcol and, when dissolution is complete, the remaining solvents are added to prepare the final concentration of drug and ratio of solvents.

Since the formulation is for parenteral use it must be sterilized. The final step is sterilization by non-heating means, preferably by membrane filtration, since the active ingredient might be subject to decomposition at autoclave temperatures.

The unexpected feature of this invention is realized in its comparison with other injectable formulations containing avermectin and milbemycin compounds. The aqueous micelle formulations provides dosages of the active compounds sufficient to eradicate substantially all internal parasites of the host animal. It was desired to obtain a parenteral formulation that also had high levels of activity against both internal and external parasites.

The formulation of the invention achieves the desired result as demonstrated in the following test:

A comparison of the efficacy of invermectin when administered to cattle by means of the formulation of the invention or by an aqueous micelle formulation shows the superiority of the formulation of the invention, particularly towards ectoparasites.

Cattle that had been infected with ticks *(Boophilus microplus)* were given a control placebo or equal doses of ivermectin via the formulation of Example 1, or an aqueous micelle formulation. The efficacy of the treatment was measured by counting the ticks in the manner described by Wharton *et. al* in *Australian Journal of Agricultural Research 21* pages 985—1006 (1970). The prolonged effects of the drug were observed by counting the ticks several times over a 4-week period with a constant natural exposure to tick infestation.

The test showed that, to a statistically significant degree, both formulations had fewer ticks than the controls. At the end of the trial there were statistically significantly more ticks on the micelle-treated cattle than on the cattle treated with the formulation of the invention, thus demonstrating greater efficacy and duration of efficacy for the instant formulation.

The following examples are provided in order that the invention might be more fully understood.

Example 1

To prepare an ivermectin injectable solution containing 10 mg of active ingredient per ml of solution the following ingredients were used:

| Ivermectin | 1.0% W/V |
| Glycerol Formal | 40.0% V/V |
| Propylene Glycol | q.s. 100.0% V/V |

The ivermection was dissolved in either the glycerol formal, the propylene glycol or a mixture of the solvents. When the dissolution was complete the volume was adjusted to the final desired volume. The final solution was sterilized by membrane filtration and packaged aseptically providing 10 mg of ivermectin per ml of solution.

Example 2

To prepare an ivermectin injectable solution containing 10 mg of active ingredient per ml of solution, the following ingredients were used:

| Ivermectin | 1.0% W/V |
| Water for Injection | 10.0% V/V |
| Propylene Glycol | q.s. 100.0% V/V |

The ivermectin was dissolved in a part of the propylene glycol. The water for injection was added in such a manner that precipitation of the ivermectin was avoided and the final volume was adjusted with propylene glycol to the desired final concentration. The solution was membrane filtered to sterilize it and packaged aseptically.

**Claims for the Contracting states: BE, CH, DE, FR, GB, IT, LI, LU, NL, SE**

1. An endoparasiticidal and ectoparasiticidal parenteral formulation comprising 0.1 to 20% W/V of an avermectin or milbemycin compound dissolved in a 10:90 to 90:10 V/V mixture of glycerol formal and propylene glycol or in a 95:5 to 80:20 V/V mixture of propylene glycol and water.

2. A formulation as claimed in Claim 1 in which the avermectin or milbemycin compound is ivermectin.

3. A formulation as claimed in Claim 2 containing 1% W/V of ivermectin dissolved in 40:60 V/V mixture of glycerol formal and propylene glycol.

4. A formulation as claimed in Claim 2 containing 1% W/V of ivermectin dissolved in 90:10 V/V mixture of propylene glycol and water.

5. A method of preparing a formulation as claimed in any one of Claims 1 to 3 comprising dissolving the avermectin or milbemycin compound in a sufficient quantity of glycerol formal, propylene glycol or a mixture thereof, then adjusting the solvent concentration to the desired level.

6. A method of preparing a formulation as claimed in any one of Claims 1, 2 and 4, comprising dissolving the avermectin or milbemycin compound in a sufficient quantity of propylene glycol to dissolve it, adding water and if necessary adjusting the concentration to the desired level with propylene glycol.

**Claims for the Contracting State: AT**

1. A method of preparing an endoparasiticidal and ectoparasiticidal parenteral formulation comprising 0.1 to 20% W/V of an avermectin or milbemycin compound dissolved in a 10:90 to 90:10 V/V mixture of glycerol formal and propylene glycol or in a 95:5 to 80:20 V/V mixture of propylene glycol and water, in which the avermectin or milbemycin compound is dissolved in a sufficient quantity of glycerol formal, propylene glycol or a mixture thereof, and the solvent concentration is then adjusted to the desired level.

2. A method of preparing an endoparasiticidal and ectoparasiticidal parenteral formulation comprising 0.1 to 20% W/V of ivermectin dissolved in a 10:90 to 90:10 V/V mixture of glycerol formal and propylene glycol or in a 95:5 to 80:20 V/V mixture of propylene glycol and water, in which the ivermectin is dissolved in a sufficient quantity of glycerol formal, propylene glycol or a mixture thereof, and the solvent concentration is then adjusted to the desired level.

3. A method as claimed in Claim 1 or 2, comprising dissolving the avermectin or milbemycin compound in a sufficient quantity of propylene glycol to dissolve it, adding water and if necessary adjusting the concentration to the desired level with propylene glycol

4. A method as claimed in Claim 2, in which the product contains 1% W/V of ivermectin dissolved in an 40:60 V/V mixture of glycerol formal and propylene glycol.

5. A method as claimed in Claim 2, in which the product contains 1% W/V of ivermectin dissolved in an 90:10 V/V mixture of propylene glycol and water.

6. A method as claimed in Claim 3, in which the avermectin or milbemycin compound is ivermectin and the product contains 1% W/V of ivermectin dissolved in 90:10 V/V mixture of propylene glycol and water.

**Patentansprüche für die Vertragsstaaten: BE, CH, DE, FR, GB, IT, LI, LU, NL, SE**

1. Endoparasitizide und ectoparasitizide parenterale Formulierung, welche 0,1 bis 20% G/V einer Avermectin- oder Milbemycinverbindung, gelöst in einer Mischung aus 10:90 bis 90:10 V/V Glycerinformal und Propylenglykol oder in einer Mischung aus 95:5 bis 80:20 V/V Propylenglykol und Wasser, umfaßt.

2. Formulierung, wie in Anspruch 1 beansprucht, worin die Avermectin- oder Milbemycinverbindung Ivermectin ist.

3. Formulierung, wie in Anspruch 2 beansprucht, welche 1% G/V Ivermectin, gelöst in einer Mischung aus 40:60 V/V Glycerinformal und Propylenglykol, enthält.

4. Formulierung, wie in Anspruch 2 beansprucht, welche 1% G/V Ivermectin, gelöst in einer Mischung aus 90:10 V/V Propylenglykol und Wasser, enthält.

5. Verfahren zur Herstellung einer Formulierung, wie in einem der Ansprüche 1 bis 3 beansprucht, welches das Lösen der Avermectin-oder Milbemycinverbindung in einer hinreichenden Menge Glycerinformal, Propylenglykol oder einer Mischung davon und danach das Einstellen der Konzentration der Lösung auf den gewünschten Grad umfaßt.

6. Verfahren zur Herstellung einer Formulierung, wie in einem der Ansprüche 1, 2 und 4 beansprucht, welches das Lösen der Avermectin-oder Milbemycinverbindung in einer hinreichenden Menge Propylenglykol zu ihrer Auflösung, die Zugabe von Wasser und, falls nötig, die Einstellung der Konzentration auf den gewünschten Grad mit Propylenglykol umfaßt.

**Patentansprüche für den Vertragsstaat: AT**

1. Ein Verfahren zur Herstellung einer endoparasitiziden und ektoparasitiziden Parenteralformulierung, enthaltend 0,1 bis 20% (Gew./Vol.) einer Avermectin- oder Milbemycinverbindung, gelöst in einem 10:90- bis 90:10- (Vol./Vol.) Gemisch aus Glycerinformal und Propylenglykol, oder in einem 95:5- bis 80:20-(Vol./Vol.) Gemisch aus Propylenglykol und Wasser, wobei die Avermectin- oder Milbeymcinverbindung in einer ausreichenden Menge von Glycerinformal, Propylenglykol, oder einem Gemisch davon, gelöst, und die Lösungsmittelkonzentration dann auf den gewünschten Wert eingestellt wird.

2. Ein Verfahren zur Herstellung einer endoparasitiziden und ektoparasitiziden Parenteralformulierung, enthaltend 0,1 bis 20% (Gew./Vol.) von Ivermectin, gelöst in einem 10:90- bis 90:10-(Vol./Vol.) Gemisch aus Glycerinformal und Propylenglykol, oder in einem 95.5- bis 80:20-(Vol./Vol.) Gemisch aus Propylenglykol und Wasser, wobei das Ivermectin in einer ausreichenden Menge von Glycerinformal, Propylenglykol, oder einem Gemisch davon, gelöst, und die Lösungsmittelkonzentration dann auf den gewünschten Wert eingestellt wird.

3. Ein Verfahren wie in Anspruch 1 oder 2 beansprucht, umfassend das Lösen der Avermectin- oder Milbemycinverbindung in einer ausreichenden Menge von Propylenglykol, um die Verbindung aufzulösen, das Zugeben von Wasser und erforderlichenfalls das Einstellen der Konzentration auf den gewünschten Wert mit Propylenglykol.

4. Ein Verfahren wie in Anspruch 2 beansprucht, wobei das Produkt 1% (Gew./Vol.) von Ivermectin, gelöst in einem Gemisch aus Glycerinformal und Propylenglykol im Verhältnis von etwa 40:60 (Vol./Vol.), enthält.

5. Ein Verfahren wie in Anspruch 2 beansprucht, wobei das Produkt 1% (Gew./Vol.) von Ivermectin, gelöst in einem Gemisch aus Propylenglykol und Wasser im Verhältnis von etwa 90:10 (Vol./Vol.), enthält.

6. Ein Verfahren wie in Anspruch 3 beansprucht, wobei die Avermectin- oder Milbemycinverbindung Ivermectin ist, und das Produkt 1% (Gew./Vol.) von Ivermectin, gelöst in einem Gemisch aus Propylenglykol und Wasser im Verhältnis 90:10 (Vol./Vol.) enthält.

**Revendications pour les Etats Contractants: BE, CH, DE, FR, GB, IT, LI, LU, NL, SE**

1. Formulation parentérale endoparasisticide et ectoparasiticide comprenant 0,1 à 20% en poids/ volume d'un composée avermectine ou milbémycine dissous dans un mélange à 10:90—90:10 V/V de glycérol-formal et de propylène-glycol ou dans un mélange à 95:5—80:20 V/V de propylène-glycol et d'eau.

2. Formulation selon la revendication 1, dans laquelle le composé avermectine ou milbémycine est l'invermectine.

3. Formulation selon la revendication 2, contenant 1% en poids/volume d'ivermectine dissoute dans un mélange à 40:60 V/V de glycérol-formal et de propylène-glycol.

4. Formulation selon la revendication 2, contenant 1% en poids/volume d'ivermectine dissoute dans un mélange à 90:10 V/V de propylène-glycol et d'eau.

5. Procédé de préparation d'une formulation selon l'une quelconque des revendications 1 à 3, comprenant la dissolution du composé avermectine ou milbémycine dans une quantité suffisante de glycérol-formal, de propylène-glycol ou d'un mélange de ceux-ci, puis l'ajustement de la concentration de solvant au niveau souhaité.

6. Procédé de préparation d'une formulation selon l'une quelconque des revendications 1, 2 et 4, comprenant la dissolution du composé avermectine ou milbémycine dans une quantité suffisant de propylène-glycol pour le dissoudre, l'addition d'eau et si nécessaire l'ajustement de la concentration au niveau souhaité avec du propylène-glycol.

**Revendications pour l'Etat Contractant: AT**

1. Procédé de préparation d'une formulation parentérale endoparasiticide et ectoparasiticide comprenant 0,1 à 20% en poids/volume d'un composé avermectine ou milbémycine dissous dans un mélange à 10:90—90:10 V/V de glycérol-formal et de propylène-glycol ou dans un mélange à 95:5—80:20 V/V de propylène-glycol et d'eau, dans lequel on dissout le composé avermectine ou milbémycine dans une quantité suffisante de glycérol-formal, de propylène-glycol ou d'un mélange de ceux-ci, et on ajuste ensuite la concentration du solvant au niveau souhaité.

2. Procédé de préparation d'une formulation parentérale endoparasiticide et ectoparasiticide comprenant 0,1 a 20% en poids/volume d'ivermectine dissoute dans un mélange à 10:90—90:10 V/V de glycérol-formal et de propylène-glycol ou dans un mélange à 95:5—80:20 V/V de propylène-glycol et d'eau, dans lequel on dissout l'ivermectine dans und quantité suffisante de glycérol-formal, de propylène-glycol ou d'un mélange de ceux-ci, et on ajuste ensuite la concentration du solvant au niveau souhaité.

3. Procédé selon la revendication 1 ou 2, comprenant la dissolution du composé avermectine ou milbémycine dans une quantité suffisante de propylène-glycol pour le dissoudre, l'addition d'eau et si nécessaire l'ajustement de la concentration au niveau souhaité avec du propylène-glycol.

4. Procédé selon la revendication 2, dans lequel le produit contient 1% en poids/volume d'ivermectine dissoute dans un mélange à environ 40:60 V/V de glycérol-formal et de propylène-glycol.

5. Procédé seon la revendication 2, dans lequel le produit contient 1% en poids/volume d'ivermectine dissoute dans un mélange à environ 90:10 V/V de propylène-glycol et d'eau.

6. Procédé selon la revendication 3, dans lequel le composé avermectine ou milbémycine est l'ivermectine et le produit contient 1% en poids/volume d'ivermectine dissoute dans un mélange à 90:10 V/V de propylène-glycol et d'eau.